# EUROPEAN PATENT APPLICATION

(11) **EP 4 378 505 A1**
(43) Date of publication of application: **05.06.2024**
(21) Application number: 22211019.9
(22) Date of filing: 02.12.2022
(51) Int. Cl.: A61M 15/06

(54) **SYSTEM AND APPARATUS**

(71) Applicant: JT International SA, 1202 Geneva (CH)
(72) Inventor: MONTICONE, Pier Paolo, 1218 Le Grand-Saconnex (CH)
(74) Representative: Appleyard Lees IP LLP

(57) **Abstract**

According to the present disclosure there is provided a system for use in an aerosol inhalation session, the system comprising: an aerosol generating device; and a processor configured to: receive a sensory stimulus response signal from a user during the aerosol inhalation session in which the user uses the aerosol generating device; and control the aerosol inhalation session based on the sensory stimulus response signal.

## Description

The present disclosure relates to a system, in particular a system for use in an aerosol inhalation session. The present disclosure also relates to an apparatus, in particular an apparatus for use in an aerosol inhalation session. The present disclosure also relates to a method, in particular a method of an aerosol inhalation session.

### Background

Sensory stimulus response signals may be used to indicate the impact of inhalable materials consumption on a user. During an aerosol inhalation session, a user uses an aerosol generating device, which includes intermittent use over a period of time as the uses takes "puffs" on the aerosol generating device during the aerosol inhalation session. During the aerosol inhalation session, the user may consume inhalable materials from the aerosol. It is understood that inhalable materials affect the senses of the user. The user may consume inhalable materials in an inappropriate manner, which may result in an unpleasant or unexpected user experience.

It is the object of the invention to overcome at least some of the above referenced problems, or problems discussed elsewhere.

### Summary

According to the present disclosure there is provided a system for use in an aerosol inhalation session, an apparatus for use in an aerosol inhalation session, and a method of an aerosol inhalation session, including the features as set out in the appended claims.

According to one aspect, there is provided a system for use in an aerosol inhalation session, the system comprising: an aerosol generating device; and a processor configured to: receive a sensory stimulus response signal from a user during the aerosol inhalation session in which the user uses the aerosol generating device; and control the aerosol inhalation session based on the sensory stimulus response signal.

In this way, the effect of aerosol inhalation on the user can be determined or estimated from the sensory stimulus response signal, and the aerosol inhalation session can be controlled based on the sensory stimulus response signal. Highly advantageously, by controlling the aerosol inhalation session based on the sensor stimulus response signal, user experience is improved.

The aerosol generating device may be referred to as a "nicotine delivery device". The processor may be integral to, or separate to, the aerosol generating device. The user may use the aerosol generating device when (i.e., synchronously) the evoked potential signal is initiated or received, or, alternatively, may use the aerosol generating device at any other time during the aerosol inhalation session.

The processor may include software or firmware running on the processor. The processor may be incorporated in a remote device such as a mobile phone. The processor may be provided by a dedicated hardware. The processor may be provided in the aerosol generating device.

Controlling the aerosol inhalation session may include controlling any one or more parameters of the aerosol inhalation session. This may include controlling operation or function of the aerosol generating device, such as controlling the (level or amount of) generation of aerosol by the aerosol generating device, or may include controlling power supplied to one or more heaters of the aerosol generating device. Additionally, or alternatively, controlling the aerosol inhalation session may include providing output to a user of the aerosol generating device.

In one example, the processor is configured to: store a sensory stimulus response signal profile and/or a threshold sensory stimulus response signal; compare the received sensory stimulus response signal with the profile and/or the threshold; and control the aerosol inhalation session based on the comparison.

By controlling the aerosol inhalation session based on a comparison between a received signal and a profile and/or threshold, user experience is improved as it can be ensured that the user properly consume aerosol during the aerosol inhalation session. Moreover, the profile or threshold sensory response signal may correspond to a preferred or optimised aerosol consumption for a particular user. In this way, user experience is improved.

In one example, the processor is configured to control the aerosol inhalation session by adjusting and/or terminating the aerosol inhalation session where the comparison indicates that the received sensory stimulus response signal differs from the profile and/or exceeds the threshold.

In this way, the aerosol inhalation session may be adjusted or terminated to avoid inappropriate aerosol consumption or an unpleasant user experience. In one example, the received sensory stimulus response may differ from the profile and/or exceed the threshold by a predetermined, or threshold, amount.

In one example, the sensory stimulus response signal is an evoked potential signal.

Use of an evoked potential signal as the sensory stimulus response signal is particularly advantageous as an evoked potential signal can be used to accurately indicate the effect of aerosol consumption, in particular nicotine consumption, on the user. Evoked potential signals can be accurately correlated with nicotine consumption.

An evoked potential (or "evoked response") is a term used to refer to the electrical potential in a specific pattern recorded from a specific part of the nervous system, especially the brain, of a human or other animals following presentation of a stimulus. Evoked potentials may be used to monitor a subject by detecting drug-related sensory alterations.

In one example, the evoked potential signal is an auditory and/or visual evoked potential signal.

Auditory and visual evoked potential signals can be accurately correlated with nicotine consumption. Advantageously, auditory evoked potential signals can be initiated using audio generating devices as a stimulator unit, such as headphones, use of which is widespread. Furthermore, auditory evoked potential signals can be initiated using natural stimuli, thus avoiding the need for the user to wear or operate an audio generating device. Furthermore, visual evoked potentials can be initiated using a display device, such as an extended reality (XR) headset, use of which is increasingly prevalent. Similarly, visual evoked potential signals can be initiated using natural stimuli, again avoiding the need for the user to wear or operate a display device.

In one example, the processor is configured to control the aerosol inhalation session by controlling operation of the aerosol generating device.

In this way, the delivery of aerosol to the user can be controlled directly. This provides a system with improved control of the user experience of the user. In this way, delivery of aerosol to the user can be controlled directly at the aerosol generating device, in contrast to requesting that the user change their consumption of aerosol.

In one example, the processor is configured to control the aerosol inhalation session by controlling an output to a user. In one example, the output to the user is an output through a user interface (Ul). In one example, the output to the user is an output in an extended reality environment.

In this way, the delivery of aerosol to the user can be controlled indirectly. The session may be controlled by controlling, or providing, an output to the user. The output to the user may be a notification, warning, alert, message, or the like. User satisfaction is thereby improved, due to increased user awareness of their aerosol consumption and/or aerosol generating device usage.

Extended reality is a term referring to all real-and-virtual combined environments and human-machine interactions generated by computer technology and wearables. For example, extended reality (XR) includes augmented reality (AR), mixed reality (MR), and virtual reality (VR). The extended reality environment may otherwise be referred to as an "extended reality inhalation environment". It is highly advantageous to provide output in an extended reality environment as user engagement is more likely and user recognition of the output can be established by monitoring eye movement. Furthermore, an XR headset can function as a stimulator unit for initiating the sensory stimulus response signal. Providing output in an extended reality environment may mean providing output whilst the user is in, or is interacting with, an extended reality environment.

In one example, the system is configured to store data related to the sensory stimulus response signal for use in control of the aerosol inhalation session. The processor may be configured to collect said data.

In this way, storing such data enables improvements aerosol inhalation session control algorithms. Furthermore, storing such data enables updating of sensory stimulus response signal profiles and threshold sensory stimulus response signals, thus improving and maintaining user experience over time as the user uses an aerosol generating device. Storing data may comprise collecting statistics for improving control algorithms, usage of the device, and other applications.

In one example, the system further comprising a wearable device. In one example, the wearable device is an audio headset or an extended reality (XR) headset. In one example, the wearable device is configured to provide the sensory stimulus response signal to the processor.

Advantageously, the wearable device can be used to initiate a sensory stimulus response signal (e.g., functioning as a stimulator unit) and also may facilitate the provision of output to the user. Furthermore, the wearable device may enable the user to access the extended reality environment.

In one example, the system comprises a brain-processor interface (BPI).

The BPI may otherwise be referred to as a brain-computer interface (BCI). A BPI is a device or system that measures activity of the central nervous system (CNS) and converts it into a sensor stimulus response signal (e.g., an electrical output). In this way, the sensory stimulus response signal, and, in particular, evoked potential signals, can be obtained and used by the system to control the aerosol inhalation session based on the sensory stimulus response signal. The BPI may be comprised in the wearable device.

The BPI may transmit the sensory stimulus response signal, in particular evoked potential signals, to the processor via wireless communication means, such as Bluetooth, WiFi or NFC.

In one example, the sensory stimulus response signal is initiated by a stimulator unit.

The user response to aerosol consumption, specifically the user reaction to nicotine consumption, can be determined from the user response or reaction to stimulation provided by the stimulator unit. Evoked potentials may be initiated by stimulus provided by the stimulator unit.

In one example, the stimulator unit is provided at a remote device and/or at the aerosol generating device. In one example, the remote device is a wearable device. In one example, the wearable device is an audio headset or an extended reality (XR) headset. In one example, the wearable device is configured to provide the sensory stimulus response signal to the processor.

Advantageously, the remote device may be a device that the user is using, such as an audio generating device or display device. The stimulator signal may be more accurate when using a remote device. Alternatively, or additionally, providing the stimulator unit at the aerosol generating device may avoid the need for an additional remote device, or, in combination, suitable stimulation signals may be generated depending on the circumstances.

In one example, the sensory stimulus response signal is initiated by a natural stimulus, the system further comprising a natural stimulus sensing unit.

In this way, a stimulator unit need not be provided, thus reducing the size, cost, and/or complexity of the system.

According to one aspect, there is provided an apparatus for use in an aerosol inhalation session, the apparatus comprising: a processor configured to: receive a sensory stimulus response signal during the aerosol inhalation session in which a user uses an aerosol generating device; and control the aerosol inhalation session based on the sensory stimulus response signal.

In this way, the apparatus may be used with an existing or pre-provided aerosol generating device to control the aerosol inhalation session. The effect of aerosol inhalation on the user can be determined or estimated from the sensory stimulus response signal, and the aerosol inhalation session can be controlled based on the sensory stimulus response signal. Highly advantageously, by controlling the aerosol inhalation session based on the sensor stimulus response signal, user experience is improved.

In one example, the processor is configured to interact with an aerosol generating device and/or a remote device.

In this way, the apparatus can control the aerosol inhalation session and/or provide output to the user by interacting with the aerosol generating device and/or the remote device. In one example, the apparatus may comprise the remote device, such as an extended reality headset, or any device having a processor configured to interact with an aerosol generating device.

According to one aspect, there is provided a method of an aerosol inhalation session, the method comprising: receiving a sensory stimulus response signal during the aerosol inhalation session in which a user uses an aerosol generating device; and controlling the aerosol inhalation session based on the sensory stimulus response signal.

Further advantages, objectives and features of the present invention will be described, by way of example only, in the following description with reference to the figures. In the figures, like components in different embodiments can exhibit the same reference symbols.

### Brief Description of the Drawings

Examples of the present disclosure will now be described with reference to the accompanying drawings.
Figure 1 shows a system;
Figure 2 shows a schematic view of an aerosol generating device;
Figure 3 shows a first example of a setup for obtaining an evoked potential signal;
Figure 4 shows a second example of a setup for obtaining an evoked potential signal;
Figure 5 shows a schematic view of an apparatus; and
Figure 6 shows general methodology principles.

### Detailed Description

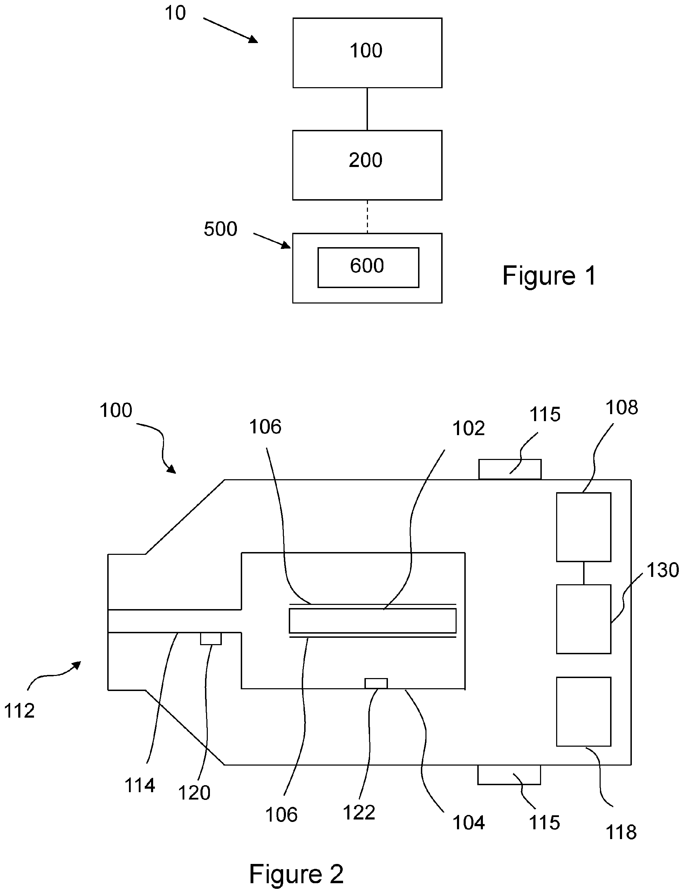

Figure 1 shows a system 10. The system 10 is for use in an aerosol inhalation session. The system 10 comprises an aerosol generating device 100 and a processor 200. The processor 200 is configured to receive a sensory stimulus response signal from a user during the aerosol inhalation session in which the user uses the aerosol generating device. The processor 200 is configured to control the aerosol inhalation session based on the sensory stimulus response signal. It should be noted that Figure 1 just shows a flow of the sensory stimulus response signal. The processor 200 may be included into the aerosol generating device 100.

In this way, closed-loop control of the aerosol inhalation session is provided based on sensory stimulus response signals from the user.

The aerosol generating device 100 may otherwise be referred to as a "vaping device" or "nicotine delivery device".

During the aerosol inhalation session, a user uses the aerosol generating device 100, which includes intermittent use over a period of time as the uses draws on, or takes "puffs" from, the aerosol generating device 100 during the aerosol inhalation session. During the aerosol inhalation session, the user may consume nicotine from the aerosol generated by the aerosol generating device 100. Consumption of nicotine affects the senses of the user. A sensory stimulus response signal provides an indication of the user's response to a sensory stimulus. The includes the user's (i.e., the user's bodily) response to the sensory stimulating effects of the consumed aerosol. The sensory stimulus response signal provides an indication of the effects of nicotine consumption on the user. For example, the sensory stimulus response signal may provide an indication of if the user is having a predetermined reaction to nicotine consumption. Additionally, the sensory stimulus response signal may be used to estimate that the user may have a predetermined reaction to nicotine at a future time during the aerosol inhalation session. The sensory stimulus response signal may be received from a user of the aerosol generating device 100. The sensory stimulus response signal may allow the processor 200 to monitor the effect of nicotine consumption on the user.

In a specific example, the sensory stimulus response signal is an evoked potential signal. However, in general, any signal provided by the user which indicates a user's response to a sensory stimulus may be used. For example, the sensory stimulus response signal may include pulse rate, pulse pattern, breathing rate, sweating, mouth moisture content, skin conductivity, electroencephalography (EEG), electrocardiogram (ECG), measures of facial muscle activity and/or a measure of pupil dilation. The sensory stimulus response signal may be a physiological response. Additionally, in general, any neural signal provided by the user may be used. A neural signal is preferred as neural signals can be accurately correlated with nicotine consumption.

The sensory stimulus response signal may be received at any time during the aerosol inhalation session. The sensory stimulus response signal may be received (or initiated, as initiation and reception may take place synchronously) whilst the user is using the aerosol generating device 100, i.e., as the user consumes aerosol from the aerosol generating device 100. Additionally, or alternatively, the sensory stimulus response signal may be received (or initiated) before the user uses the aerosol generating device 100 for the first time during the aerosol inhalation session. This provides for an initial measurement of the sensory stimulus response signal absent the effects of nicotine consumption. Additionally, or alternatively, the sensory stimulus response signal may be received (or initiated) after the user has used the aerosol generative device 100 (e.g., after the user has consumed nicotine) during the aerosol inhalation session. This provides for a measurement of the sensory stimulus response signal which may be impacted by the consumption of nicotine by the user.

The processor 200 may thereby be configured to "automatically" control (which may include adjusting or terminating) the aerosol inhalation session based on the sensory stimulus response signal. That is, the processor 200 may be configured to automatically control the aerosol inhalation session without user input or user control of the session. This may be referred to as closed-loop control of the aerosol inhalation session.

The processor 200 may include software or firmware running on the processor 200. The processor 200 may be incorporated in a remote device such as a mobile phone. The processor 200 may be provided by a dedicated hardware. The processor 200 may be provided in the aerosol generating device 100.

As will be described in greater detail below, the processor 200 may form part of the aerosol generating device 100 or may be separate to the aerosol generating device 100, for example may form part of a remote device.

Figure 2 shows a schematic cross-sectional view of an aerosol generating device. The aerosol generating device 100 is suitable for receiving a consumable 102 therein. For example, the aerosol generating device 100 may include a chamber 104 in which the consumable 102 is received.

The invention is not limited to the specific aerosol generating device 100 or consumable 102 described herein. That is, the description of the aerosol generating device 100 and consumable 102 is provided for illustrative purposes only. The skilled person will appreciate that alternative constructions of aerosol generating devices and consumables will be compatible with the present invention.

A consumable 102 comprises an aerosol substrate. The term aerosol substrate is a label used to mean a medium that generates an aerosol or vapour when heated. Aerosol substrate might be interpreted as an aerosol precursor. In one example, aerosol substrate is synonymous with smokable material, aerosol generating substrate and aerosol generating medium. Aerosol substrate includes materials that provide volatilized components upon heating, typically in the form of vapor or an aerosol. Aerosol substrate may be a non-tobacco-containing material or a tobacco-containing material. Aerosol substrate may, for example, include one or more of tobacco per se, tobacco derivatives, expanded tobacco, reconstituted tobacco, tobacco extract, homogenized tobacco or tobacco substitutes. Aerosol substrate also may include other, non-tobacco, products, which, depending on the product, may or may not contain nicotine. Aerosol substrate may comprise one or more humectants, such as glycerol or propylene glycol.

The aerosol generating device 100 may comprise one or more heaters 106 configured to provide heat to the consumable 102, in use.

In one example, the consumable 102 contains a liquid and the one or more heaters comprises a heating element, such as a coil, a ceramic heater, a flat resistive heater, a mesh heater, a MEMS heater, or the like, configured to aerosolise the liquid for inhalation. A liquid delivery element or mechanism, such as a porous material, a capillary system, and/or valve, may transfer the liquid to the heating element, in use. In some examples, the aerosolised liquid may pass through a solid substrate within the aerosol generating device 100. In other examples, the consumable 102 may comprise a solid aerosol substrate.

In one example, the aerosol generating device 100 comprises a nebulizing engine, such as a vibrating mesh, to generate an aerosol from a liquid with or without heating thereof.

The aerosol generating device 100 may comprise a mouthpiece 112 through which a user draws on the aerosol generating device 100 to inhale generated aerosol. The mouthpiece 112 includes a vent or channel 114 that is connected to a region close to the consumable 102 for passage of any generated aerosol from the consumable 102, during use. For example, the channel 114 may extend between an opening in the mouthpiece 112 and the chamber 104 in which the consumable 102 is at least partially receivable. The mouthpiece 112 is arranged such it may be received in a user's mouth in use. In other examples, a mouthpiece 112 is not required and a portion of the consumable 102 may protrude from the aerosol generating device 100. In this example, protruded portion of the consumable 102 may work as mouthpiece.

The aerosol generating device 100 may comprise a control unit 108 (or control circuitry) for electronic management of the device. The control unit 108 may include a PCB or the like (not shown). The control unit 108 is configured to control the one or more heaters 106. The control unit 108 may form at least a part of the processor 200.

The aerosol generating device 100 may comprise an activation input sensor 118. The activation input sensor 118 may be a button, a touchpad, or the like for sensing a user's input, such as a tap or swipe. In other examples, the activation input sensor 118 comprises a consumable sensor configured to detect if a consumable 102 has been inserted into the aerosol generating device 100. For example, the input sensor 118 may comprise an authenticity detector that is configured to detect if an authentic consumable 102 has been inserted into the aerosol generating device 100. Additionally, or alternatively, the user input may also comprise an inhalation action by a user.

The aerosol generating device 100 may comprise a puff sensor 120 (otherwise known as an inhalation sensor). The puff sensor 120 is configured to detect an inhalation action (or puff) by a user on the aerosol generating device 100. In one example, the puff sensor 120 comprises a microphone or a flow sensor configured to an airflow within the chamber 104 and/or an airflow channel extending from the chamber 104 through the mouthpiece 112 to an inhalation outlet thereof, the airflow being associated with a user's inhalation action. In other examples, the puff sensor 120 is configured to detect a change in pressure indicative of a beginning of an inhalation action on the aerosol generating device by the user. In this case, the puff sensor 120 may be located anywhere on the aerosol device 100 in which there would be a change in pressure due to an inhalation action of the user. In one example, the puff sensor 120 is located in the channel 114 between the chamber 104 and the mouthpiece 112 of the aerosol generating device 100. The puff sensor 120 may also detect the end of an inhalation action by the user. For example, the puff sensor 120 may be configured to detect a further change in pressure due to the end of an inhalation action of a user.

The aerosol generating device 100 may include one or more temperature sensors 122 configured to directly or indirectly measure the temperature of the consumable 102 in the aerosol generating device 100. The one or more temperature sensors 122 may comprise a temperature sensor, such as a thermocouple or thermistor, configured to be located within or adjacent to the consumable 102 when it is received in the aerosol generating device 100. For example, the one or more temperature sensors 122 may be located within the chamber 104 of the aerosol generating device 100. In other examples, the temperature of the consumable 102 may be indirectly measured by the use of thermal imaging sensors. In further other example, the heater 106 itself works as a temperature sensor if the heater 105 has PTC (Positive Temperature Coefficient) or NTC (Negative Temperature Coefficient) characteristic.

The aerosol generating device 100 may include a power supply (not shown) such as a battery. The power supply may provide the aerosol generating device 100 with electrical energy providing a voltage in the range of 3 V and 4.2 V. In a preferred embodiment the voltage source is a lithium-ion secondary battery delivering a value of 3.7 V. Such a voltage source is particularly advantageous for a modern aerosol generating device in view of rechargeability, high energy density and large capacity.

The aerosol generating device 100 may comprise a controller 130. The controller 130 is connected to the control unit 108. The controller 130 is configured to receive data from the control unit 108. In particular, the controller 130 is configured to receive data from the control unit 108 relating to various sensors/inputs (such as the activation input sensor 118, puff sensor 120 and/or temperature sensor 122) of the aerosol generating device 100. The controller 130 may form at least a part of the processor 200.

The controller 130 and the control unit 108 may be integral with each other. In one example, a single component performs the function of the control unit 108 and controller 130. In other examples, the control unit 108 and the controller 130 are distinct components.

Referring back to Figure 1, further operation of the system 10 will be described.

In an example, the processor 200 is configured to store a sensory stimulus response signal profile and/or a threshold sensory stimulus response signal.

A sensory stimulus response signal profile may be a profile of sensory stimulus response signals for the user which is expected, or desired, during the aerosol inhalation session. The sensory stimulus response signal profile may be a pre-recorded profile, or predetermined profile, based on prior use of the aerosol generating device 100. Additionally, or alternatively, the sensory stimulus response signal profile may be a default profile for a user.

A threshold sensory stimulus response signal may be a threshold of the sensory stimulus response signal beyond which further nicotine consumption may be considered inappropriate, undesired, or expected to result in a negative experience for the user.

In an example, the processor 200 is configured to compare the received sensory stimulus response signal with the sensory stimulus response signal profile and/or the threshold sensory stimulus response signal.

The comparison may be based on the sensory stimulus response signal at the time in which it is received. That is, the comparison may look to determine whether the received sensory stimulus response matches the sensory stimulus response signal profile for that particular time point. Additionally, the comparison may look to determine whether the received sensory stimulus response signal is under, close to, or exceeds the threshold. In this way, it can be monitored whether the user is operating the aerosol generating device 100 in an appropriate manner.

In an example, the processor 200 is configured to control the aerosol inhalation session based on the comparison.

In this way, closed-loop control of the aerosol inhalation session (in particular, the nicotine consumption of the user) can be achieved. Control of the aerosol inhalation session may be achieved in numerous ways, as described in greater detail below, including, but not limited to, controlling generation of aerosol by the aerosol generating device 100, controlling power supplied to the one or more heaters 106, and/or providing a notification, warning, or message to the user.

In an example, the processor 200 is configured to control the aerosol inhalation session by adjusting and/or terminating the aerosol inhalation session where the comparison indicates that the received sensory stimulus response signal differs from the sensory stimulus response signal profile and/or exceeds the threshold sensory stimulus response signal.

Such a construction is particularly advantageous as closed-loop control of the aerosol inhalation session based on sensory stimulus response signals is provided for.

The processor 200 may establish that the received sensory stimulus response signal differs from the sensory stimulus response signal profile and/or exceeds the threshold sensory stimulus response signal by an amount greater than a predetermined amount. The predetermined amount may be an amount considered inappropriate or likely to result in an unpleasant user experience. The predetermined amount may differ based on the specific user of the aerosol generating device 100 and/or time in the aerosol inhalation session.

The processor 200 may be configured to adjust the aerosol inhalation session by controlling generation of aerosol by the aerosol generating device 100, controlling power supplied to the one or more heaters 106, or adjust an output to the user at a display device which the user is viewing or interacting with (e.g., adjust or change a notification, warning, or message output to the user).

The processor 200 may be configured to terminate the aerosol inhalation session by terminating the generation of aerosol by the aerosol generating device 100, terminating the supply of power to the one or more heaters 106, and/or terminate display of output to the user at a display device which the user is viewing or interacting with (e.g., terminate an extended reality environment which the user is viewing or interacting with, which may include replacing the extended reality environment with a notification, warning, or message).

In an example, the sensory stimulus response signal is an evoked potential signal.

An evoked potential (or "evoked response") is a term used to refer to the electrical potential in a specific pattern recorded from a specific part of the nervous system, especially the brain, of a human or other animals following presentation of a stimulus. Evoked potentials may be used to monitor a subject by detecting drug-related sensory alterations.

Using the evoked potential signal, it is possible to determine (or estimate) the effect of nicotine consumption on the user. Using the evoked potential signal, the effect may be determined (or estimated) independently of the user's characteristics (e.g., weight, age, and/or sex).

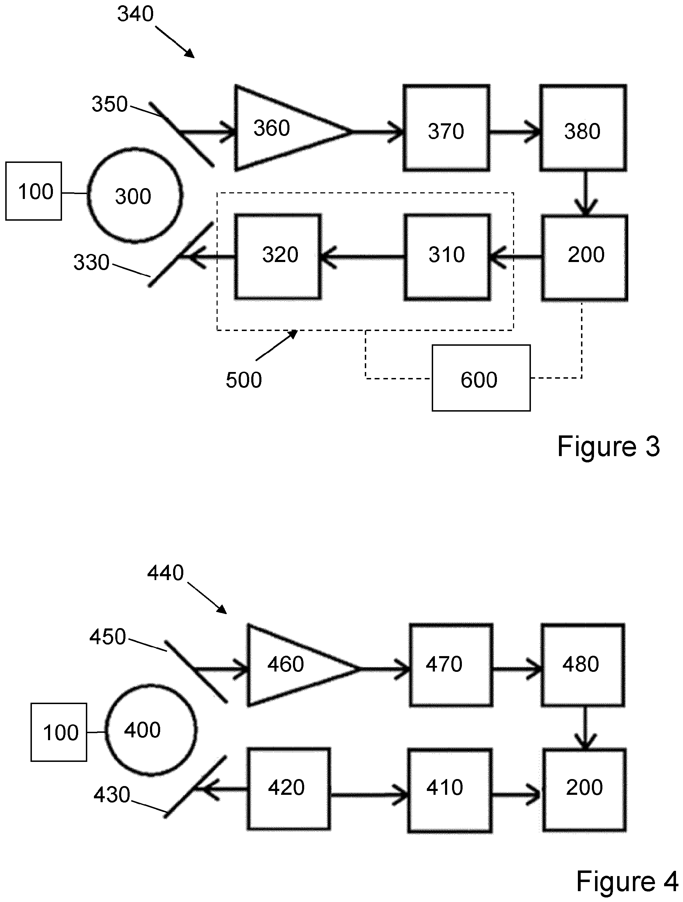

Referring to Figures 3 and 4, block diagrams of setups for obtaining the evoked potential signal are shown. The setups may each, or both, be incorporated in the system 10. It will be appreciated that other setups may be suitable for obtaining the evoked potential signal, and the invention is not limited to the specific setups shown and described. Instead, the setups are shown and described as an example of how evoked potential signals may be obtained.

Referring to Figure 3, first example of a setup for obtaining an evoked potential signal is shown. A user of an aerosol generating device 100 is indicated at 300. The user 300 is using the aerosol generating device 100 during the aerosol inhalation session.

The processor 200 provides a trigger 310 (such as an initiating signal) to a stimulator unit 320. The stimulator unit 320 is configured to provide a stimulus based on the trigger 310, or stimulating output, to the user 300. Many different stimuli modalities and types may be used. As an example, the stimulus may be an auditory stimulus, such as a pure tone. As an example, the stimulus may be a visual stimulus, such as a light flash. The stimulus is provided to the user 300 at a stimulating site 330.

The stimulus provided to the user 300 initiates a sensory stimulus response signal from the user 300 of the aerosol generating device 100, specifically, an evoked potential signal.

In an example, the user 300 is provided with a brain-processor interface (BPI) 340. The brain-processor interface may also be referred to using the term "brain-computer interface (BCI) 340". BPIs, or BCIs, are in general well known. The BPI is a communication pathway between the brain's electrical activity and the processor 200 or a computer. In this way, the sensory stimulus response signal from the user 300 may be detected, transmitted to the processor 200 or computer, to be received by the processor 200 or computer.

A recorder 350, or "recording site", records or detects the sensory stimulus response signal, for transmission to the processor 200.

In an example, an amplifier 360 amplifies the recorded sensory stimulus response signal and a filter 370 filters the amplified recorded sensory stimulus response signal. An analogue-to-digital converter (ADC) 380 converts the amplified and filtered recorded sensory stimulus response signal to a digital format, and transmits the digital sensory stimulus response signal to the processor 200. The processor 200 receives the sensory stimulus response signal.

The various components described above (processor 200, trigger 310, stimulator unit 320, recorder 350, amplifier 360, filter 370, ADC 380) may be arranged in the system 10 in a number of different ways. In one example, the recorder 350, amplifier 360, filter 370 and ADC 380 are provided at, i.e., incorporated in, the BPI 340, and the processor 200, trigger 310 and stimulator unit 320 are provided at a mobile phone or at the aerosol generating device 100. In another example, the recorder 350, amplifier 360, filter 370 are provided at the BPI 340, the processor 200 and trigger 310 are provided at a mobile phone or at the aerosol generating device 100, and the stimulator unit 320 is provided at a wearable device such as an audio headset (e.g., headphones) or a wearable device (e.g., a wearable display device) such as an XR headset.

Referring to Figure 4, a second example of a setup for obtaining an evoked potential signal is shown. In the setup of Figure 4, the sensor stimulus response signal is initiated by a natural, such as a stimulus from the environment of the user.

A user of an aerosol generating device 100 is indicated at 400. The user 400 is using the aerosol generating device 100 during the aerosol inhalation session.

A natural stimulus sensing unit 410 is provided to provide input to the processor 200. The natural stimulus sensing unit 410 is configured to sense a natural stimulus (e.g., a stimulus not produced by the system 10). The natural stimulus sensing unit 410 may comprise, or be, a microphone, camera and/or light sensor. It will be appreciated that a suitable sensor should be chosen based on the stimuli modalities and type of stimulus that it is desired to sense. The natural stimulus sensing unit 410 provides input to the processor 200 when a natural stimulus is sensed.

A natural stimulus is indicated at block 420. The natural stimulus may be a visual stimulus, such as a light flash or colour change, or an auditory stimulus, such as a sound, a repeating sound, an alarm, or the like. The natural stimulus is provided to the user 400 at a stimulating site 430.

As described above in relation to Figure 3, the natural stimulus provided to the user 400 initiates a sensory stimulus response signal from the user 400 of the aerosol generating device 100, specifically, an evoked potential signal.

In an example, the user 400 is provided with a brain-processor interface (BPI) 440. The brain-processor interface may also be referred to using the term "brain-computer interface (BCI) 440". BPIs, or BCIs, are in general well known. The BPI is a communication pathway between the brain's electrical activity and the processor 200 or a computer. In this way, the sensory stimulus response signal from the user 400 may be detected, transmitted to the processor 200 or computer, to be received by the processor 200 or computer.

A recorder 450, or "recording site", records or detects the sensory stimulus response signal, for transmission to the processor 200.

In an example, an amplifier 460 amplifies the recorded sensory stimulus response signal and a filter 470 filters the amplified recorded sensory stimulus response signal. An analogue-to-digital converter (ADC) 480 converts the amplified and filtered recorded sensory stimulus response signal to a digital format, and transmits the digital sensory stimulus response signal to the processor 200. The processor 200 receives the sensory stimulus response signal.

The various components described above (processor 200, natural stimulus sensing unit 410, recorder 450, amplifier 460, filter 470, ADC 480) may be arranged in the system 10 in a number of different ways. In one example, the recorder 450, amplifier 460, filter 470 and ADC 480 are provided at, i.e., incorporated in, the BPI 440, and the processor 200 and natural stimulus sensing unit 410 are provided at a mobile phone or at the aerosol generating device 100. In another example, the recorder 450, amplifier 460, filter 470 are provided at the BPI 440, the processor 200 is provided at a mobile phone or at the aerosol generating device 100, and the natural stimulus sensing unit 410 is provided at a wearable device such as an audio headset (e.g., headphones) or a wearable device (e.g., a wearable display device) such as an XR headset.

In an example, the evoked potential signal is an auditory and/or visual evoked potential signal.

An auditory evoked potential signal is an evoked potential signal initiated by an auditory stimulus. As described above in relation to Figures 3 and 4, the auditory stimulus may be generated by a stimulator unit 320 or may be a natural auditory stimulus sensed by a natural stimulus sensing unit 410.

A visual evoked potential signal is an evoked potential signal initiated by a visual stimulus. As described above in relation to Figures 3 and 4, the visual stimulus may be generated by a stimulator unit 320 or may be a natural visual stimulus sensed by a natural stimulus sensing unit 410.

Use of auditory and/or visual evoked potential signals is particularly advantageous as they can be accurately correlated with nicotine consumption and the effects thereof on the user. User experience can thereby be improved, by controlling the aerosol inhalation session based on the auditory and/or visual evoked potential signal.

Referring back to Figure 1, further features of the system 10 are described.

In an example, the processor 200 is configured to control the aerosol inhalation session by controlling operation of the aerosol generating device 100. That is, the processor 200 may be configured to control the aerosol inhalation session based on the sensory stimulus response signal by controlling operation of the aerosol generating device 100.

In this way, where a predetermined reaction of the user is detected, or anticipated, the processor 200 can control the aerosol inhalation session by controlling the aerosol generating device 100 to mitigate or prevent the predetermined reaction.

The processor 200 may be configured to control the operation of the aerosol generating device 100 by controlling generation of aerosol by the aerosol generating device 100 and/or controlling power supplied to the one or more heaters 106. In an example, where the sensory stimulus response signal indicates that inappropriate nicotine consumption has occurred, the processor 200 may control the aerosol generating device 100 to reduce the generation and delivery of aerosol for consumption by the user. Furthermore, the processor 200 may control the aerosol generating device 100 to prevent or terminate the delivery of aerosol to the user. Such control may be referred to as closed-loop control of the aerosol inhalation session.

In an example, the processor 200 is configured to control the aerosol inhalation session by controlling an output to a user. The output to the user may be an output in an extended reality environment.

The output to the user may be a notification, warning, alert, message, or the like. The output may inform the user of excess or unsafe nicotine consumption, or that continued operation of the aerosol generating device 100 may lead to inappropriate nicotine consumption. The output may be provided through a user interface viewable by the user, or provided by the aerosol generating device, for example an audible output or a tactile output. The user interface may be provided by a wearable device, as described herein.

Providing the output in an extended reality environment may be particularly advantageous as the user may be distracted in the extended reality environment and thus less aware of their nicotine consumption. The extended reality environment may be a virtual reality environment, e.g., whilst the user is wearing a wearable device such as an XR or VR headset.

In an example, the system 10 is configured store data related to the sensory stimulus response signal for use in control of the aerosol inhalation session.

The system 10 may be provided with, or connected to, a memory for storing said data. Said data may include statistics. Said data may be used to update, adjust, and/or improve control algorithms for controlling operation of the aerosol generating device 100. For example, said data may be used to adjust sensory stimulus response signal profiles and/or thresholds, which may subsequently be used in control of the aerosol inhalation session to adjust delivery of aerosol to the user.

In an example, the system 10 further comprises a wearable device 500. For example, the wearable device 500 may be an audio headset or an extended reality (XR) headset.

As described above in relation to Figures 3 and 4, the wearable device 500 may provide the stimulator unit 320 and/or the natural stimulus sensing unit 310. Furthermore, the wearable device 500 may be configured to provide output to the user, such as a notification, warning, alert, message, or the like.

In an example, the wearable device 500 is configured to provide the sensory stimulus response signal to the processor 200. That is, the wearable device 500 may detect a sensory stimulus response signal from the user and provide said signal to the processor 200.

In an example, the system 10 comprises a brain-processor interface (BPI) 600. The BPI may otherwise be referred to as a brain-computer interface (BCI). Operation of the BPI 600 is as described above in relation to Figures 3 and 4. The BPI 600 may be connected to the processor 200. That is, sensory stimulus response signals from the user are detected by the BPI 600 and provided to the processor 200. The BPI 600 may provide the sensory response signals to the processor 200 wirelessly, via wireless connection means such as Bluetooth, WiFi or NFC.

The BPI 600 may be comprised in the wearable device 500. Alternatively, the BPI 600 and wearable device 500 may be distinct (i.e., separate) components.

The BPI 600 may be non-invasive. In this way, the user may readily use and remove the BPI 600 when desired or when required during the aerosol inhalation session. Alternatively, the BPI 600 may be partially invasive or invasive. In this way, the accuracy of sensory stimulus response signals may be improved.

In an example related to Figure 3 described above, the sensory stimulus response signal is initiated by a stimulator unit 320. The stimulator unit 320 may form part of the system 10, or may be separate to the system 10. That is, the system 10 may be used in conjunction with an existing stimulator unit 320.

As described above, many different stimuli modalities and types may be used. However, in specific examples, the stimulator unit 320 may be an auditory stimulator unit configured to generate an auditory stimulus (such as a pure tone), or may be a visual stimulator unit configured to generate a visual stimulus (such as a light flash), for provision to the user.

In an example, the stimulator unit is provided at a remote device and/or at the aerosol generating device. In an example, the remote device is a wearable device 500, such as an audio headset or XR headset, as described above.

In other words, the remote device and/or aerosol generating device may be configured to provide the stimulus. The remote device may include, or be, a mobile phone running an application for providing (e.g., generating) the stimulus. The mobile phone may be incorporated in the wearable device 500.

In an example related to Figure 4 described above, the sensory stimulus response signal is initiated by a natural stimulus, and the system 10 further comprises a natural stimulus sensing unit 410.

A natural stimulus may be a stimulus provided by, or in, the environment of the user - i.e., not provided by the system 10. The natural stimulus sensing unit 410 may comprise, or be, one or more microphones, cameras and/or light sensors.

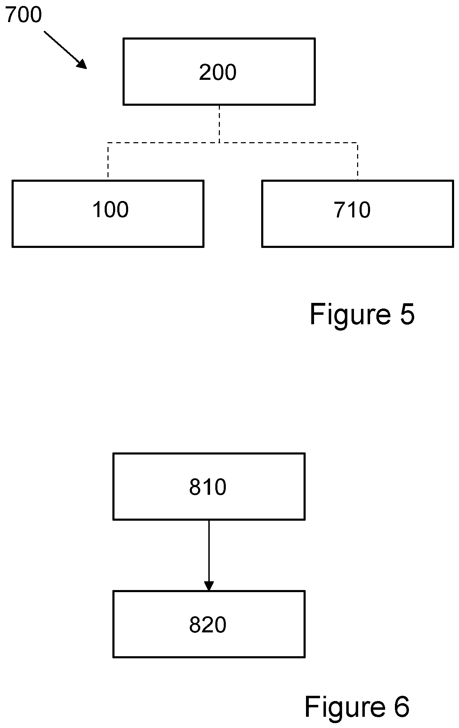

Referring to Figure 5, an apparatus 700 for use in an aerosol inhalation session is shown. The apparatus comprises a processor 200 configured to: receive a sensory stimulus response signal during the aerosol inhalation session in which a user uses an aerosol generating device; and control the aerosol inhalation session based on the sensory stimulus response signal. The processor 200 may be configured to interact with an aerosol generating device 100 and/or a remote device 710. The remote device 710 may be a wearable device 500, as described above.

It will be appreciated that the apparatus 700 may be provided in absence of an aerosol generating device 100 and/or remote device, and configured to interact with an aerosol generating device and/or remote device 710. That is, the apparatus may be retrofit in an existing system 10 as described above.

The apparatus 700 may comprise any of the features of system 10 described above, as desired or as appropriate.

Referring to Figure 6, a method of an aerosol inhalation session is shown. Step 810 comprises receiving a sensory stimulus response signal during the aerosol inhalation session in which a user uses an aerosol generating device. Step 820 comprises controlling the aerosol inhalation session based on the sensory stimulus response signal.

The method may include any or all features of the system 10 or apparatus 700 described above, as desired or as appropriate.

Although preferred embodiments have been shown and described, it will be appreciated by those skilled in the art that various changes and modifications might be made without departing from the scope of the invention. One or more of the steps described above may be optional and not performed in systems and methods without departing from the scope of the invention. The scope of the invention is as defined in the appended claims.

## Claims

1. A system for use in an aerosol inhalation session, the system comprising:
an aerosol generating device; and
a processor configured to:
receive a sensory stimulus response signal from a user during the aerosol inhalation session in which the user uses the aerosol generating device; and
control the aerosol inhalation session based on the sensory stimulus response signal.

2. The system according to claim 1, wherein the processor is configured to:
store a sensory stimulus response signal profile and/or a threshold sensory stimulus response signal;
compare the received sensory stimulus response signal with the profile and/or the threshold; and
control the aerosol inhalation session based on the comparison.

3. The system according to claim 2, wherein the processor is configured to control the aerosol inhalation session by adjusting and/or terminating the aerosol inhalation session where the comparison indicates that the received sensory stimulus response signal differs from the profile and/or exceeds the threshold.

4. The system according to any one of the preceding claims, wherein the sensory stimulus response signal is an evoked potential signal.

5. The system according to claim 4, wherein the evoked potential signal is an auditory and/or visual evoked potential signal.

6. The system according to any one of the preceding claims, wherein the processor is configured to control the aerosol inhalation session by controlling operation of the aerosol generating device.

7. The system according to any one of the preceding claims, wherein the processor is configured to control the aerosol inhalation session by controlling an output to a user.

8. The system according to any one of the preceding claims, wherein the system is configured to store data related to the sensory stimulus response signal for use in control of the aerosol inhalation session.

9. The system according to any one of the preceding claims, the system further comprising a wearable device.

10. The system according to any one of the preceding claims, wherein the system comprises a brain-processor interface (BPI).

11. The system according to any one of the preceding claims, wherein the sensory stimulus response signal is initiated by a stimulator unit.

12. The system according to claim 11, wherein the stimulator unit is provided at a remote device and/or at the aerosol generating device.

13. The system according to any one of the preceding claims, wherein the sensory stimulus response signal is initiated by a natural stimulus, the system further comprising a natural stimulus sensing unit.

14. An apparatus for use in an aerosol inhalation session, the apparatus comprising:
a processor configured to:
receive a sensory stimulus response signal during the aerosol inhalation session in which a user uses an aerosol generating device; and
control the aerosol inhalation session based on the sensory stimulus response signal.

15. A method of an aerosol inhalation session, the method comprising:
receiving a sensory stimulus response signal during the aerosol inhalation session in which a user uses an aerosol generating device; and
controlling the aerosol inhalation session based on the sensory stimulus response signal.
